# EUROPEAN PATENT SPECIFICATION

(11) **EP 0 906 100 B1**
(45) Date of publication and mention of the grant of the patent: **28.01.2004**
(21) Application number: 97906125.6
(22) Date of filing: 25.02.1997
(51) Int. Cl.: A61K 31/495

(54) **USE OF 5-HT 1A? RECEPTOR ANTAGONISTS FOR THE TREATMENT OF URINARY INCONTINENCE**
VERWENDUNG VON ANTAGONISTEN DES 5-HTIA REZEPTORS ZUR BEHANDLUNG VON HARNINKONTINENZ
UTILISATION D'ANTAGONISTES DES RECEPTEURS 5-HT 1A? POUR LE TRAITEMENT DE L'INCONTINENCE URINAIRE

(30) Priority: 28.02.1996 IT MI960378
(43) Date of publication of application: 07.04.1999
(73) Proprietor: RECORDATI S.A., 6830 Chiasso (CH); RECORDATI INDUSTRIA CHIMICA E FARMACEUTICA S.p.a., 20148 Milano (IT)
(72) Inventor: LEONARDI, Amedeo, I-20154 Milano (IT); TESTA, Rodolfo, I-20060 Vignate (IT)
(74) Representative: McKelvey, Ian Edward
(86) International application number: PCT/EP1997/000897
(87) International publication number: WO 1997/031637

(56) References cited:
- EP-A- 0 512 755
- EP-A- 0 558 245
- WO-A-95/33743
- WO-A-96/05817
- J.PHARMACOL.EXP.THER, vol. 262, no. 1, 1992, pages 181-189, XP000675356 A. LECCI ET AL.: "Involvement of 5-hydroxytryptamine 1A receptors in the modulation of micturition reflexes in the anaesthetized rat." cited in the application

## Description

The invention relates to compositions and methods for treating certain disorders of the lower urinary tract in mammals, including humans, using serotonin 5-HT_{1A} receptor antagonist compounds which exert their inhibitory effects via both pre-synaptic (somatodendritic) and post-synaptic antagonism.

In mammals, micturition is a complex process which requires the integrated actions of the bladder, its internal and external sphincters, the musculature of the pelvic floor, and neurological control over these muscles at three levels (in the bladder wall or sphincter itself, in the autonomic centres of the spinal cord, and in the central nervous system at the level of the pontine micturition centre in the brainstem (pons) under the control of the cerebral cortex). Micturition results from contraction of the detrusor muscle, which consists of interlacing smooth muscle fibres under parasympathetic autonomic control from the sacral spinal cord. A simple voiding reflex is formed by sensory nerves for pain, temperature, and distension that run from the bladder to the sacral cord. However, sensory tracts from the bladder also reach the pontine micturition centre, resulting in the generation of nerve impulses that normally suppress the sacral spinal reflex arc controlling bladder emptying. As a result, normal micturition is initiated by voluntary suppression of cortical inhibition of the reflex arc and by relaxation of the muscles of the pelvic floor and the external sphincter. Finally, the detrusor muscle contracts and voiding occurs.

Functional abnormalities of the lower urinary tract, e.g., dysuria, incontinence, and enuresis, are common in the general population. Dysuria includes urinary frequency, nocturia, and urgency, and may be caused by cystitis, prostatitis or benign prostatic hypertrophy (which affects about 70% of elderly males), or by neurological disorders. Incontinence syndromes include stress incontinence, urgency incontinence, and overflow incontinence. Enuresis refers to the involuntary passage of urine at night or during sleep.

Prior to the present invention, treatment of neuromuscular disorders or the lower urinary tract has involved administration of compounds which act directly on the bladder muscles, such as flavoxate, a spasmolytic drug also active on the pontine micturition centre, or anticholinergic compounds such as oxybutynin. The use of α₁-adrenergic receptor antagonists for the treatment of benign prostatic hypertrophy is also common. However, treatments that involve direct inhibition of the pelvic musculature (including the detrusor muscle) may have unwanted side effects such as incomplete voiding or accommodation reflex paralysis, tachycardia and dry mouth. Thus, it would be preferable to utilize compounds which act via the peripheral or central nervous system, for example to affect the sacral spinal reflex arc and/or the inhibition pathways of the pontine micturition centre in a manner that restores normal functioning of the micturition mechanism.

Lecci et al (*J. Pharmacol. Exp. Therapeutics*, 262, 181, 1992) describe the effects of the 5-HT_{1A} receptor ligands 8-hydroxy-2-(di-N-propylamino)-tetralin (8-OH-DPAT) and 1-(2-methoxyphenyl)-4-[4-(2-phthalimido)-butyl]-piperazine (NAN 190) on micturition reflexes in the anaesthetized rat. 8-OH-DPAT (an agonist) stimulated the supraspinal micturition reflex originating from the pontine micturition centre, while NAN-190 inhibited the supraspinal micturition reflex. The authors concluded that spinal and supraspinal 5-HT_{1A} receptors modulate the supraspinal micturition reflex in this system. The present inventors, however, have found that the efficacy of NAN-190 and other 5-HT_{1A} receptor ligands in inhibiting the supraspinal micturition reflex is directly correlated to their relative binding affinities for the α₁-adrenergic receptor, rather than to their affinities, if any, for the 5-HT_{1A} receptor which called into question the relevance of the effects correlated to 5-HT_{1A} receptor activity for lower urinary tract disorders. Finally, as discussed below, NAN-190 is considered a partial 5-HT_{1A} receptor agonist rather than a complete or "true" antagonist. Therefore, prior to the present invention, the use of true 5-HT_{1A} receptor antagonists to treat urinary tract disorders was unknown.

At least two functionally distinct types of the 5-HT_{1A} receptor have been identified, and these are designated "pre-synaptic" (or somatodendritic) and "post-synaptic". Pre-synaptic receptors are present on 5-HT-producing neurons and are involved in autoregulation of 5-HT release; their activation causes physiological changes including hyperphagia, hypothermia (in the mouse), bradycardia and hypotension. Post-synaptic receptors are widely distributed throughout the mammalian brain and are coupled to potassium channels and adenylate cyclase; their activation leads to "5-HT behavioural syndrome", hypothermia (in the rat), and elevation of plasma corticotropin levels. Beyond the differences in their anatomical distribution and functioning, pre-synaptic and post-synaptic receptors can be distinguished by the differential activity profiles of different 5-HT_{1A} receptor ligands. For example, full agonists such as 8-OH-DPAT and 5-carboxytryptamine have agonist activity on both pre-synaptic and post-synaptic receptors. By contrast, partial agonists such as huspirone, ipsapirone, spiroxantine, urapidil, NAN-190 and BMY 7378 have agonist activity on pre-synaptic receptors and antagonist activity on post-synaptic receptors. Finally, some compounds exhibit antagonistic activity on both pre-synaptic and post-synaptic receptors. These last compounds, true antagonists of the serotoninergic 5-HT_{1A} receptor, are useful in the invention.

The invention provides the use of a compound which
(a) hinds to a cloned human 5-HT_{1A} receptor with an affinity of at least 10⁻⁷ M,
(b) hinds to a rat 5-HT_{1A} receptor with an affinity at least 50 times greater than the affinity with which the compound hinds to a rat α₁-adrenergic receptor, and
(c) exhibits 5-HT_{1A} receptor antagonist activity on both mouse pre-synaptic and rat post-synaptic 5-HT_{1A} receptors,
or of a stereoisomer, hydrate, solvate or pharmaceutically acceptable salt of such a compound, for the preparation of a medicament for the treatment of neuromuscular disorders of the lower urinary tract in mammals.

Compounds useful in the practice of the invention preferably bind to 5-HT_{1A} receptors with an affinity (K₁) of at least 10⁻⁸ M. Expressing their 5-HT_{1A} receptor antagonist activity (at both pre-synaptic and post-synaptic sites) as a function of dose, the compounds may have ID₅₀ values of from 1 to 2000 µg/Kg, and preferably of from 1 to 200 µg/Kg.

As the compounds useful in the practice of the invention bind to 5-HT_{1A} receptors with an affinity at least 50 times greater, and preferably 100 times greater, than they bind to α₁-adrenergie receptors, they have α₁-adrenergie receptor binding constants in the micromolar range or weaker.

Medicaments prepared according to the invention are suitable for the treatment of neuromuscular disorders of the lower urinary tract, particularly those involving micturition, such as dysuria, incontinence, and enuresis. Without wishing to be bound by theory, it is believed that administration of 5-HT_{1A} receptor antagonists prevents unwanted activity of the sacral reflex arc and/or cortical mechanisms that control micturition. Thus it is contemplated that a wide range of lower urinary tract disorders can be treated using the compounds of the invention.

The compounds of the present invention may be formulated into liquid dosage forms with a physiologically acceptable carrier, such as phosphate buffered saline or deionized water. The pharmaceutical formulation may also contain excipients, including preservatives and stabilizers, which are well-known in the art. The compounds can be formed into solid oral or non-oral dosage units such as tablets, capsules, powders, and suppositories, and may additionally include excipients, including without limitation lubricants, plasticizers, colorants, absorption enhancers, bactericides, and the like. Administration of medicaments prepared using true 5-HT_{1A} receptor antagonist compounds, their stereoisomers, pharmaceutically acceptable salts, hydrates or solvates, may be achieved by any effective route, including oral, enteral, intravenous, intramuscular, subcutaneous, transdermal, transmucosal (including rectal and buccal), and inhalation routes. Preferably, an oral or transdermal route is used (i.e., via solid or liquid oral formulations, or skin patches, respectively).

An effective amount of the medicament is an amount that results in measurable amelioration of at least one symptom of the disorder. This effective amount can be determined by experimentation known in the art, such as by establishing a matrix of dosages and frequencies and comparing a group of experimental units or subjects to each point in the matrix. Symptoms of urinary tract disorders include urgency, frequency, urine leakage, enuresis, dysuria, hesitancy, difficulty in emptying bladder. A measurable amelioration of any symptom or parameter is determined by a physician skilled in the art or reported by the patient to the physician.

For example, a single patient may suffer from several symptoms of dysuria simultaneously, such as, for example, urgency and frequency, either or both of which may be reduced using the methods of the present invention. In the case of incontinence, any reduction in the frequency or volume of unwanted passage of urine is considered a beneficial effect of the present methods of treatment.

The amount of the agent to be administered may range from about 0.01 to about 25 mg/kg/day, preferably from about 0.1 to 10 mg/kg/day and most preferably from about 0.2-5 mg/kg/day. It will be understood that the medicament formulations of the present invention need not in themselves contain the entire amount of the agent that is effective in treating the disorder, as such effective amounts can be reached by administration of a plurality of doses.

In a preferred embodiment of the invention, N-{2-[4-(2-methoxyphenyl)-1-piperazinyl]--ethyl}-N-(2-pyridyl)-cyclohexanecarboxamide (hereinafter Compound A) is formulated in capsules or tablets each containing from 50 to 200 mg of Compound A, and is administered to a patient at a daily dose of 200 mg for relief of urinary incontinence. In another preferred embodiment of the invention, N-{2-[4-(4-indolyl)-1-piperazinyl]--ethyl}-N-(2-pyridyl)-cyclohexanecarboxamide (hereinafter Compound B) is formulated in capsules or tablets each containing from 20 to 60 mg of Compound B, and is administered to a patient at a daily dose of 60 mg for relief of urinary incontinence.

Compounds which possess the requisite pre-synaptic and post-synaptic 5-HT_{1A} antagonistic activity may be found amongst those having a protonatable nitrogen atom which is linked on one side to an aromatic or heteroaromatic ring and to a carbon chain on the other side. In addition, the protonatable nitrogen and the chain can form a ring. Compounds belonging to this general class may be tested according to the methods discussed below to determine whether they meet the other criteria for use in the invention.

Measurement of the specific binding activity of a compound towards different neuronal receptors (such as serotoninergic 5-HT_{1A} receptors, α₁- or α₂-adrenergic receptors, dopaminergic D₂ receptors, and serotoninergic 5-HT₂ receptors) may be achieved using any of a multiplicity of methods that are well known in the art, such as competitive binding to native or cloned receptors. Typically, a biological source of, for example, a 5-HT_{1A} receptor is used in which the receptor is present at a sufficiently high concentration so that labelled 5-HT or a labelled 5-HT_{1A} ligand is easily measurable. This source may comprise a mammalian tissue or fluid (either in situ or after removal) or a tissue culture cell. The target receptor may be expressed from either an endogenous gene or from a transfected receptor-encoding recombinant gene. For example, the rat hippocampus is a rich source of 5-HT_{1A} receptors. Alternatively, human 5-HT_{1A} receptor cDNA can be expressed in *E. coli* cells in culture as reported in Bertin B. et al., *J. Biol. Chem*., 267, 8200 (1992). The ability of the test compound to compete with labelled 5-HT (or a labelled 5-HT_{1A} ligand) for receptor binding is then measured, and a binding constant is calculated using Scatchard analysis or equivalent computational methods well known in the art.

It will be understood that measurements of receptor binding affinity of a particular compound may vary depending upon the source of receptor, radiolabelled ligand, and other components, as well as specific assay conditions. Thus, Compound A is included in all assays as a standardization control. That is, the values of binding affinities obtained for Compound A are compared to values reported below in Example 3, i.e. Kᵢ = 3 X 10⁻¹⁰ M for 5-HT_{1A} receptors and 3 X 10⁻⁷ M for α₁-adrenergic receptors, and the values obtained in the same assay for other test compounds are normalized proportionately.

Measurement of pre-synaptic and post-synaptic serotoninergic 5-HT_{1A} receptor antagonist activity may be achieved using neurophysiological assay methods. For example, Raphe cell firing measured electrophysiologically is used as an index of pre-synaptic 5-HT_{1A} receptor activity (VanderMaelen et al., *Brain Res*., 289, 109, 1983). In this assay, a 5-HT_{1A} receptor agonist acting at pre-synaptic somatodendritic 5-HT_{1A} receptors inhibits Raphe neuronal firing, which is detected by measuring the electrical activity of 5-HT-containing neurons. Antagonists prevent the inhibitory action of the 5-HT_{1A} receptor agonist, resulting in the maintenance of high levels of serotonin in the synaptic cleft. An alternative system for measuring pre-synaptic activity is the inhibition of hypothermia induced in mice by 8-OH-DPAT (Moser, *Eur. J. Pharmacol*., 193, 165 , 1991).

Inhibition of adenylate cyclase activity in rat hippocampal slices is used as an indicator of post-synaptic 5-HT_{1A} receptor activity (Shenker et al.,*Eur.J.Pharmacol*., 109, 427, 1985). In this assay, compounds exhibiting antagonistic activity at post-synaptic 5-HT_{1A} receptors antagonize the inhibitory effects of a 5-HT_{1A} agonist only on forskolin-stimulated adenylate cyclase activity and display no intrinsic effect on the basal activity of the enzyme. Alternative methods for measuring post-synaptic activity include inhibition of 8-OH-DPAT induced behavioural syndrome, in particular the forepaw treading symptom (Tricklebank et al., *Eur. J. Pharmacol*., 117, 15, 1985). These and other methods are reviewed in Fletcher et al., *TiPS,* 14, 441, 1993.

As discussed above, Compound A is included in all assays as a positive control; the values obtained for the pre-synaptic and post-synaptic antagonistic activity of Compound A are normalized proportionately with those disclosed below in Examples 4 and 5 (ID₅₀ for pre-synaptic = 8.5 µg/Kg; post-synaptic = 14 µg/Kg), and the values obtained for other test compounds are compared.

Once a compound is identified as possessing 5-HT_{1A} receptor antagonist activity, its pharmacological activity is confirmed using one or more animal model systems for lower urinary tract disorders. Useful animal model systems include isovolumetric rhythmic bladder voiding contractions in anaesthetized rats and cystometry in conscious rats. In the first method, the urinary bladder is catheterized, ligated, and connected with a pressure recording device. The bladder is then filled until reflex voiding contractions occur, after which the frequency and amplitude of the voiding contractions are measured. In the second method, bladder volume capacity and micturition pressure are measured one day following bladder catheterization. In the first method, the test compounds are administered intravenously prior to the measurements. Both oral and intravenous administration routes may be used in a second method. These methods are described in more detail in Examples 6 and 7 below, and were originally used to validate the predictive qualities of the true serotoninergic 5-HT_{1A} receptor antagonists for the foregoing urinary tract disorders.

5-HT_{1A} receptor antagonist compounds for use in the invention include piperazine derivatives of the general formula I

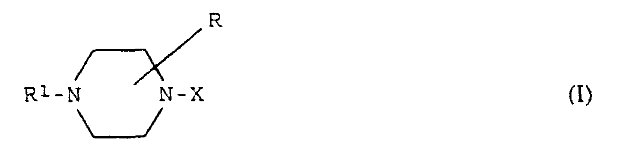

In these compounds:
R represents a hydrogen atom or a lower alkyl group;
R¹ represents an aryl, nitrogen-containing heteroaryl or nitrogen-containing bicyclic heteroaryl group; and
X represents one of the groups 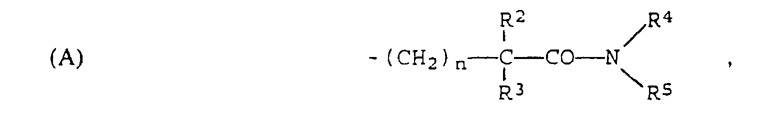 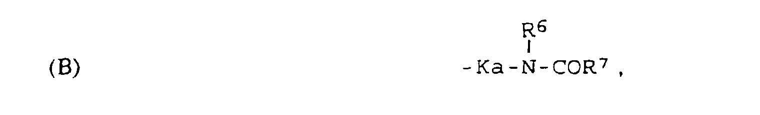 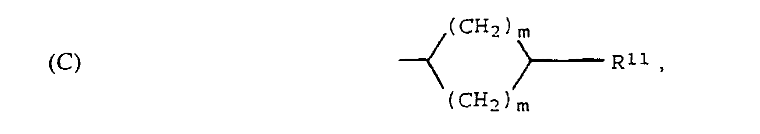 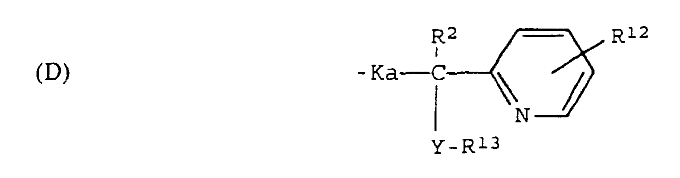
and 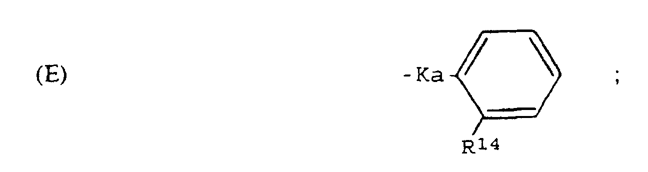
n is 1 or 2;
m is 1, 2 or 3;
R² represents a hydrogen atom or a lower alkyl group;
R³ represents an aryl or aryl(lower)alkyl group;
R⁴ represents a hydrogen atom or a C₁-C₃ alkyl group;
R⁵ represents a hydrogen atom or a C₁-C₃ alkyl, C₃-C₁₂ cycloalkyl or cycloalkyl(lower)alkyl group; or R⁴ and R⁵ together with the nitrogen atom to which they are attached represent a 1-azetidinyl, 1-pyrrolidinyl, piperidino, 1-perhydroazepinyl, morpholino, or 1-piperazinyl group, each optionally substituted by a lower alkyl, aryl or aryl(lower)alkyl group;
R⁶ represents a monocyclic or bicyclic heteroaryl group;
R⁷ represents a hydrogen atom, a lower alkyl, cycloalkyl, cycloalkenyl, cycloalkyl(lower)alkyl, aryl, aryl(lower)alkyl, heteroaryl or heteroaryl(lower)alkyl group, or a group -NR⁸R⁹ or OR¹⁰;
R⁸ represents a hydrogen atom or a lower alkyl, aryl or aryl(lower)alkyl group;
R⁹ represents a hydrogen atom or a lower alkyl, -CO-(lower)alkyl, aryl, -CO-aryl, aryl(lower)alkyl, cycloalkyl or cycloalkyl(lower)alkyl group; or R⁸ and R⁹ together with the nitrogen atom to which they are attached represent a saturated heterocyclic group which may contain an additional heteroatom;
R¹⁰ represents a lower alkyl, cycloalkyl, cycloalkyl -(lower)alkyl, aryl, aryl(lower)alkyl, heteroaryl or heteroaryl(lower)alkyl group;
R¹¹ represents an aryl or nitrogen containing heteroaryl group;
R¹² represents a hydrogen atom or a lower alkyl group;
R¹³ represents a hydrogen atom or a C₁-C₃ alkyl, C₃-C₁₂ cycloalkyl or cycloalkyl(lower)alkyl group;
R¹⁴ represents an aryl group;
Ka represents a C₂-C₄ alkylene group optionally substituted by one or more lower alkyl groups; and
Y represents a carbonyl, alkylene, hydroxyalkylene or hydroxycycloalkylene group or a group -S(O)ₒ; where o = 0 to 2.

When R¹ represents an aryl group, it preferably represents a phenyl group having a substituent in the ortho position or a 1-naphthyl group optionally substituted in the 2 or 7 positions. Examples of aryl groups R¹ are o-(lower)alkoxyphenyl, such as o-methoxyphenyl, or (lower)alkoxy substituted 1-naphthyl. When R¹ represents a bicyclic heteroaryl group, it preferably represents a 4-indolyl group.

When R⁶ represents a bicyclic heteroaryl group, both rings can contain hetero ring atom(s), or only one ring can contain hetero atom(s). In the latter instance, the group R⁶ is connected to the compound of formula (I) via the ring containing the hetero atom(s).

Examples of the heteroaryl group R⁶ include monocyclic groups containing one hetero atom, such as pyridyl (particularly 2-pyridyl); monocyclic groups containing two hetero atoms, such as thiazolyl (particularly 2-thiazolyl); and bicyclic groups containing one or two hetero atoms, such as quinolinyl or isoquinolinyl and particularly 2-quinolinyl.

When R¹¹ and R¹⁴ represent aryl groups, the preferred groups are phenyl. When R¹¹ represents a heteroaryl group it is preferably a pyridyl group, optionally substituted by one or more alkyl groups.

Methods for the preparation of the piperazine derivatives I are disclosed in the following references: GB 2230780 (EP 395313), GB 2230781 (EP 395312), GB 2248836, (EP 481744), GB 2255337, GB 2262093, WO 94/15919, WO 94/15928, WO 94/21610, WO 95/33743 and GB 2277517.

Preferred piperazine derivatives I include:
1-{4-[4-(2-methoxyphenyl)-1-piperazinyl]-3-phenylbutanoyl}-perhydroazepine,
1-{4-[4-(2-methoxyphenyl)-1-piperazinyl]-2-phenylbutanoyl}-perhydroazepine,
1-[N-cyclohexylcarbonyl-N-(2-pyridyl)-2-aminoethyl)-4-(2-methoxyphenyl)-piperazine (Compound A),
1-[N-cyclohexylcarbonyl-N-(2-pyridyl)-2-aminoethyl)-4-(4-indolyl)-piperazine (Compound B), and
1-[2-(2-biphenyl)-ethyl]-4-(2-methoxyphenyl)-piperazine,
and their pharmaceutically acceptable acid addition salts.

As used herein, aryl refers to aromatic groups having from 6 to 12 carbon atoms, such as phenyl and naphthyl. These may be substituted by one or more substituents. Preferred substituents include halogen atoms and lower alkyl, lower alkoxy, halo(lower)alkyl, nitro, cyano, amido, (lower)alkoxycarbonyl; amino; (lower)alkylamino; and di(lower)alkylamino groups. Two adjacent substituents on the aromatic ring can together to form a further fused ring, e.g. benzodioxanyl. The term halogen refers to fluorine, chlorine, and bromine. The preferred halogens are chlorine and fluorine. Examples of the preferred aryl(lower)alkyl groups include benzyl and phenethyl, optionally substituted as described above.

As used herein, heteroaryl refers to an aromatic group containing one or more hetero atoms (e.g. oxygen, nitrogen, or sulphur) and which can be monocyclic or bicyclic. The monocyclic heteroaryl group refers to an aromatic ring containing one or more nitrogen or other heteroatoms, such as pyridyl, 2-thienyl, 2-furanyl, 1-methyl-2-pyrrolyl, pyrimidinyl, pyrazinyl, oxazolyl, thiazinyl and the like. Preferred heteroaryl groups include 2-pyridyl, 3-pyridyl and 4-pyridyl groups. The heteroaryl groups may be substituted by halogen atoms or lower alkyl, lower alkoxy, halo(lower)alkyl, nitro, amino, cyano, amido, (lower)alkoxycarbunyl, (lower)alkylamino, and di-(lower)alkylamino groups. Bicyclic heteroaryl refers to phenyl rings fused with a second ring containing one or more heteroatoms. A particularly preferred heteroatom is nitrogen. Examples of the bicyclic heteroaryl groups include indazolyl, quinolinyl, isoquinolinyl and indolyl. The bicyclic heteroaryl groups can be substituted by one or more substituents. A preferred bicyclic heteroaryl group is indolyl substituted with alkoxycarbonyl groups.

The following Examples illustrate the invention. Reference is made in the Examples to the accompanying drawings, of which:
Figure 1 is a graphic illustration of a typical recorder tracing showing the effect of Compound A on volume-induced contractions of anaesthetized rats, the arrow indicating the intravenous administration of 300 µg/kg of Compound A, and
Figure 2 is a graphic illustration of a typical recorder tracing showing the effect of Compound A on cystometrographic parameters in conscious rats, the arrow indicating oral treatment of the animal with 3 mg/kg of Compound A.

### Example 1

### N-{2-[4-(2-methoxyphenyl)-1-piperazinyl]-ethyl}-N-(2-pyridyl)-cyclohexanecarboxamide . 2.66 HCl . 0.33 H₂O (Compound A)

23.5 g of 1-(2-aminoethyl)-4-(2-methoxyphenyl)-piperazine [Hexachemie-Reuil Malmaison-France] and 4.85 ml of 2-chloropyridine were stirred at 160°C in a closed reaction vessel for 10.5 hours. The reaction mixture was cooled to room temperature, dissolved in 320 ml of chloroform and washed with 1N sodium hydroxide (3 x 320 ml), followed by water (2 x 400 ml). The organic layer was dried on sodium sulphate and then evaporated to dryness under reduced pressure. The crude product was purified by column flash chromatography eluting with an ethyl acetate : 3N NH₃ in methanol 100:2 mixture affording, after evaporation of the collected fractions, 5 g of 1-[N-(2-pyridyl)-2-aminoethyl]-4-(2-methoxyphenyl)-piperazine as an oil. A sample was crystallized from ethyl acetate to give a solid melting at 89-94°C.
¹H-NMR (CDCl₃, δ)
8.08, ddd, 1H, CH at position 6 in the pyridine ring
7.40, ddd, 1H, CH at position 4 in the pyridine ring
6.80-7.05, m, 4H, 2-methoxyphenyl CHs
6.55, ddd, 1H, CH at position 5 in the pyridine ring
6.40, dd, 1H, CH at position 3 in the pyridine ring
5.10, bs, 1H, NH
3.85, s, 3H, CH₃O
3.38, dt, 2H, CH₂NH
3.00-3.15, m, 4H, piperazine CHs
2.60-2.75, m, 6H, piperazine CHs and CH₂N
D₂O addition makes NH signal appear upfield as HDO.

To a solution of 4.26 g of the compound thus prepared in 42.5 ml of tetrahydrofuran, 6.52 ml of 2.5N n-butyllithium (hexane solution) was added dropwise at -22°C. After 40 minutes stirring at -20°C, 2.21 ml of cyclohexanecarbonyl chloride was added dropwise. The reaction mixture was stirred at -20°C for 20 minutes, then at room temperature for 3.5 hours. Water was cautiously added to quench the reaction, followed by 3N sodium hydroxide. Ethyl acetate extraction followed by washing the organic layer with water, drying on sodium sulphate and evaporating the solvent to dryness under reduced pressure gave an oily crude which was purified by column flash chromatography eluting with an ethyl acetate : 3N NH₃ in methanol 100:2 mixture. Evaporation of the collected fractions afforded 5 g of the title compound as a base, which was converted into the hydrochloride by dissolution in methanol and addition of excess 2.8N HCl in diethyl ether. Evaporation to dryness of the solvents and desiccation of the solid in vacuo yielded 5.30 g of the title compound. M.p. 161-164°C.

Elemental analysis for C₂₅H₃₄N₄O₂ . 2.66 HCl . 0.33 H₂O:
calc. (%): C 57.14, H 7.16, N 10.66, Cl 17.95, H₂O 1.13
found (%): C 57.45, H 7.29, N 10.67, Cl 18.13, H₂O 1.20
¹H-NMR (D₆-DMSO, δ):
11.10-11.70, bs, 1H, NH⁺
8.58, dd, 1H, CH at position 6 in the pyridine ring
8.05, ddd, 1H, CH at position 4 in the pyridine ring
7.64, dd, 1H, CH at position 3 in the pyridine ring
7.45, dd, 1H, CH at position 5 in the pyridine ring
6.82-7.10, m, 4H, 2-methoxyphenyl CHs
5.20-5.80, bs, 2.4H, NH⁺ (remaining), H₂O
4.17, t, 2H, CH2NCO
3.79, s, 3H, CH₃O
3.00-3.75, m, 10H, CH₂N and piperazine CHs
2.15-2.35, m, 1H, cyclohexane CHCO
0.85-1.85, m, 10H, cyclohexane CHs
D₂O addition makes NH signals appear upfield as HDO.

### Example 2

### N-{2-[4-(4-Indolyl)-1-piperazinyl]-ethyl}-N-(2-pyridyl)-cyclohexanecarboxamide (Compound B)

### Step a:

### N-(2,2-Dimethoxyethyl)-N-(2-pyridyl)-cyclohexanecarboxamide

13.2 ml of a 2.5M solution of butyl lithium in n-hexane was added to a solution of 6g of 2-(2-pyridylamino)-acetaldehyde dimethyl acetal (prepared as described in Beilstein E III/IV, 22, 3871) in 40 ml of tetrahydrofuran stirred at 0°C and the resulting mixture was stirred at room temperature for 1 hour. 4.46 ml of cyclohexanecarbonyl chloride was then added dropwise over a period of 5 minutes. Stirring was continued for 5.5 hours and the reaction mixture was then evaporated to dryness *in vacuo.* The residue was purified by flash chromatography, eluting with chloroform:ethyl acetate 7:3, to afford 13.3 g of the title compound.
¹H-NMR (CDCl₃, δ):
8.48-8.54, m, 1H, CH at position 6 in the pyridine ring
7.75, ddd, 1H, CH at position 4 in the pyridine ring
7.18-7.44, m, 2H, CHs at positions 3 and 5 in the pyridine ring
4.65, t, 1H, CHCH₂
3.90, d, 2H, CH₂
3.31, s, 6H, 2 x CH₃O
2.32, tt. 1H, CH(CH₂)₂
0.80-1.85, m, 10H, cyclohexane CH₂s

### Step b:

### N-Formylmethyl-N-(2-pyridyl)-cyclohexanecarboxamide

A mixture of 1.46 g of N-(2,2-dimethoxyethyl)-N-(2-pyridyl)--cyclohexanecarboxamide, 0.05 g of 1.4-hydroquinone and 25ml of 2N HCl was stirred at 80°C for 20 minutes under a nitrogen stream. The mixture was then cooled to 0°C, diluted with 50ml of dichloromethane, and adjusted to pH 10 by addition of a 20% solution of sodium carbonate. The aqueous layer was extracted twice with dichloromethane and the combined organic layers were dried over anhydrous sodium sulphate. Evaporation to dryness *in uacuo* gave 0.94 g of the title compound, used without purification in the next reaction step.
¹H-NMR (CDCl₃, δ):
9.66, s, 1H, CHO
8.48-8.54, m, 1H, CH at position 6 in the pyridine ring
7.79. ddd, 1H, CH at position 4 in the pyridine ring
7.18-7.44, m, 2H, CHs at positions 3 and 5 in the pyridine ring
4.52, s, 2H, CH₂
2.48, tt, 1H, CH(CH₂)₂
0.80-1.95, m, 10H, cyclohexane CH₂s

### Step c:

### N-{2-[4-(4-Indolyl)-1-piperazinyl]-ethyl}-N-(2-pyridyl)-cyclohexanecarboxamide HCl . 1.25H₂O

A mixture of 0.94 g of N-formylmethyl-N-(2-pyridyl)-cyclohexanecarboxamide, 0.69 g of 1-(4-indolyl)-piperazine, 1.21 g of sodium triacetoxyborohydride, 0.44 ml of acetic acid and 30 ml of 1,2-dichloroethane was stirred at room temperature for 3 hours. The mixture was then diluted with 20 ml of water and adjusted to pH 10 by addition of a 20% solution of sodium carbonate. The aqueous layer was extracted twice with 1,2-dichloroethane and the combined organic layers were dried over anhydrous sodium sulphate. Evaporation to dryness *in vacuo* gave a crude product which was purified by flash chromatography, eluting with dichloromethane:methanol 98:2 to 95:5, to give 0.96 g of the base of the title compound. This was dissolved in 40 ml of dichloromethane and 3.8N hydrogen chloride in diethyl ether was added. The title compound precipitated and was filtered off. Yield 0.66 g. M.p. 181-187°C.

### Example 3

### Measurement of Binding of Test Compounds to 5-HT_{1A} and α₁-Adrenergic Receptors

### [³H]prazosin binding (α₁-receptors):

Rat cerebral cortices were homogenized in 50 volumes of ice-cold 50 mM Tris-HCl pH 7.4. The homogenates were centrifuged at 48,000 x g for 10 minutes, and the pellets were resuspended in the same volume of ice-cold buffer, centrifuged, and resuspended two more times. The final pellets were resuspended in 100 volumes of 50 mM Tris-HCl, pH 7.4, containing 0.1 % ascorbic acid and 10 µM pargyline. 1-ml samples were incubated for 30 min at 25°C with 0.35 nM [³H]prazosin, in the absence or presence of different concentrations (10⁻⁵ to 10⁻¹⁰ M) of the test compound. Non-specific binding was determined in the presence of 10 µM phentolamine. The incubations were terminated by rapid filtration through Whatman GF/B filters using a Brandel cell harvester, after which the filters were washed with 3x3 ml of ice-cold buffer. The radioactivity retained on the filters was determined by liquid scintillation counting. The results are shown in Table 1 below.

### [³H]8-OH-DPAT binding (5HT_{1A} receptors):

Rat hippocampi were homogenized in 50 volumes of ice-cold 50 mM Tris-HCl pH 7.4. The homogenates were centrifuged at 48,000 x g for 10 minutes, and the pellets were resuspended in the same volume of ice-cold buffer, incubated for 10 minutes at 37°C, centrifuged and resuspended two more times. The final pellets obtained were resuspended in 100 volumes of 50 mM Tris-HCl, pH 7.4, containing 0.1 % ascorbic acid and 10 µM pargyline. 1 ml samples were incubated for 30 min at 25°C with 1 nM [³H]8-OH-DPAT, in absence or presence of different concentrations (10⁻⁵ to 10⁻¹⁰ M) of the test compound. Non-specific binding was determined in the presence of 10 µM 5-HT. The incubations were terminated by rapid filtration through Whatman GF/B filters using a Brandel cell harvester, after which the filters were washed with 3 x 3 ml of ice-cold buffer. The radioactivity retained on the filters was determined by liquid scintillation counting. The results are shown in Table 1 below.

**TABLE 1**

| **Binding affinity for the 5-HT**_{**1A**} **receptor and α**_{**1**}**-adrenergic receptor.** *Data are expressed as Ki (nM).* | | |
|---|---|---|
| Compound | 5-HT_{1A} receptor | α₁-adrenergie receptor |
| Compound A | 0.3 | 295.5 |
| Compound B | 0.13 | 458.3 |
| NAN-190 | 1.9 | 4.8 |

These results indicate that Compound A and Compound B bind tightly and selectively to the 5-HT_{1A} receptor. By contrast, NAN-190 exhibits nearly equivalent binding to both receptors.

### Example 4

### Measurement of Pre-Synaptic 5-HT_{1A} Receptor Antagonist Activity

### Antagonism of hypothermia induced by 8-OH-DPAT in mice:

The antagonistic effect of 5-HT_{1A} receptor antagonists on hypothermia induced by 8-OH-DPAT was evaluated by the method of Moser (Moser, *Eur. J. Pharmacol*., 193, 165, 1991) with minor modifications.

Male CD-1 mice (28-38 g) obtained from Charles River (Italy) were housed in a climate-controlled room (temperature 22 ± 2°C; humidity 55 ± 15%) and maintained on a 12 h light/dark cycle with free access to food and water. On the day of experiment, mice were placed singly in clear plastic boxes under the same ambient conditions. Body temperature was measured by the insertion of a temperature probe (Termist TM-S, LSI) into the rectum to a depth of 2 cm. Rectal temperature was measured immediately prior to subcutaneous injection of the test compound and 30 min later. All animals then received 8-OH-DPAT (0.5 mg/kg s.c.) and their temperature was measured 30 min later. For each animal, temperature changes were calculated with respect to pretreatment values and the mean values were calculated for each treatment group.

A linear regression equation was used in order to evaluate ID₅₀ values, defined as the dose of antagonist needed to block 50% of the hypothermic effect induced by 0.5 mg/kg 8-OH-DPAT administered subcutaneously.

The results are shown in Table 2 below.

**TABLE 2**

| **Antagonistic activity for the pre-synaptic 5-HT**_{**1A**} **receptor.** | |
|---|---|
| COMPOUND | ID₅₀ (95%) C.L. in µg/kg s.c. |
| Compound A | 8.5 (5.8-12.5) |
| Compound B | 1.9 (1.0-3.7) |
| NAN-190 | not active |

These results demonstrate that Compound A and Compound B have significant pre-synaptic 5-HT_{1A} receptor antagonist activity.

### Example 5

### Measurement of Post-Synaptic 5-HT_{1A} Receptor Antagonist Activity

### Inhibition of forepaw treading induced by 8-OH-DPAT in rats:

The inhibitory effect of 5-HT_{1A} receptor antagonists on the forepaw treading induced in rats by subcutaneous injection of 8-OH-DPAT was evaluated by the method of Tricklebank (Tricklebank et al., *Eur.J. Pharmacol*., 117: 15, 1985) with minor modifications.

Male Sprague-Dawley rats (150-175 g) obtained from Charles River (Italy), were housed in a climate-controlled room and maintained on a 12 h light/dark cycle with free access to food and water. On the day of experiment, rats were placed singly in clear plastic boxes. Reserpinised rats were treated with reserpine, 1 mg/kg s.c., 18-24 h before the test. For evaluation of antagonistic activity, compounds were i.p. or s.c. administered 16 min before 8-OH-DPAT (1 mg/kg s.c.). Observation sessions of 30 s duration began 3 min after treatment with the agonist and were repeated every 3 min over a period of 15 min.

The appearance of the forepaw treading symptom induced by post-synaptic stimulation of the 5HT_{1A} receptors was noted, and its intensity was scored using a ranked intensity scale in which: 0 = absent, 1 = equivocal, 2 = present and 3 = intense. Behavioural scores for each treated rat were accumulated over the time course (5 observation periods) and expressed as mean values of 8-10 rats.

A linear regression equation was used in order to evaluate ID₅₀ values, defined as the dose of antagonist needed to block 50% of the forepaw treading intensity induced by 1 mg/kg 8-OH-DPAT administered subcutaneously.

The results are shown in Table 3 below.

**TABLE 3**

| Compound | NORMAL RATS ID₅₀ µg/Kg | RESERPINIZED RATS ID₅₀ µg/Kg |
|---|---|---|
| Compound A (s.c.) | 14 (12-16) | 8.5 (5.8-12.5) |
| NAN-190 ( i. p. ) | 700 (500-1000) | 2000 (1600-2400) |

These results demonstrate that Compound A exhibits significant post-synaptic 5-HT_{1A} receptor antagonist activity. NAN-190, by contrast, is much less active. Taken together, the bioassays for pre-synaptic and post-synaptic antagonist activity are effective for identifying compounds that exhibit both activities at levels that render them useful in treating urinary tract disorders.

### Example 6

### Effect of 5-HT_{1A} Receptor Antagonists on Volume-Induced Rhythmic Bladder Voiding Contractions in Anaesthetized Rats

Female Sprague Dawley rats weighing 225-275 g (Crl: CD° BR, Charles River Italia) were used. The animals were housed with free access to food and water and were maintained on a forced 12 h alternating light-dark cycle at 22-24°C for at least one week, except during the experiment. The activity on the rhythmic bladder voiding contractions was evaluated according to the method of Dray (*J. Pharmcol. Methods*, 13:157, 1985), with some modifications as in Guarneri (*Phannacol. Res*., 27:173, 1993). Briefly, rats were anaesthetized by subcutaneous injection of 1.25 g/kg (5 ml/kg) urethane, after which the urinary bladder was cathetized via the urethra using PE 50 polyethylene tubing filled with physiological saline. The catheter was tied in place with a ligature around the external urethral orifice and was connected with conventional pressure transducers (Statham P23 ID/P23 XL). The intravesical pressure was displayed continuously on a chart recorder (Battaglia Rangoni KV 135 with DC1/TI amplifier). The bladder was then filled via the recording catheter by incremental volumes of warm (37°C) saline until reflex bladder voiding contractions occurred (usually 0.8-1.5 ml). Two parameters were recorded from the cystometrogram: the frequency of voiding contractions, calculated by counting the number of peaks/15 min of observation, and the mean amplitude of these contractions (mean height of the peaks in mmHg) in the same time period. For intravenous (i. v. ) injection of bioactive compounds, a PE 50 polyethylene tubing filled with physiological saline was inserted into the jugular vein.

Bioactivity was assessed in individual animals (using 6-10 rats per dose), by recording the number and height of the peaks for 15 min after drug injection and comparing them with those previously recorded for 15 min after the intravenous administration of vehicle alone. In the evaluation of the mean amplitude of peaks after treatment, only the results from the cystometrograms showing a reduction in the frequency of contractions of ≤50% were utilized. The statistical significance of changes in frequency and amplitude before and after treatment was assessed by Student's t test for paired data. Changes showing a probability P<0.01 were considered significant.

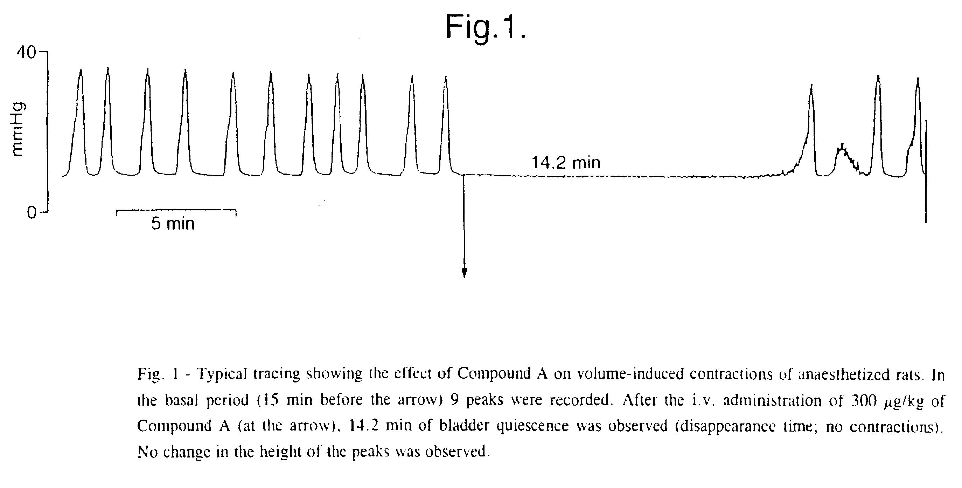

An all-or-none criterion was also used to compare bioactivity in terms of ED₅₀ values. Rats showing a treatment-related change of ≥30% relative to the basal value were considered to be positive. Quantal dose-response curves and ED₅₀ values were evaluated by the method of Bliss (*J. Pharm. Pharmacol*., 11:192, 1938). In addition, since most compounds produced an effect that was relatively rapid in onset and led to a complete cessation of bladder contractions (as shown in Figure 1), bioactivity was conveniently estimated by measuring the duration of bladder quiescence (i.e., the duration of time during which no contractions occurred). To compare the potency of the tested compounds in inhibiting the frequency of the bladder voiding contractions, equieffective doses producing 10 minutes of disappearance time (ED₁₀ₘᵢₙ) were computed by means of least square linear regression analysis.

The rapid distension of the urinary bladder in urethane-anaesthetized rats produced a series of rhythmic bladder voiding contractions whose characteristics have been described (Maggie et al., *Brain Res.,* 380, 83, 1986; Maggi, et al., *J. Pharmacol. Exp. Ther*., 230, 500, 1984). The frequency of these contractions is related to the sensory afferent arm of reflex micturition and to the integrity of the micturition centre, while their amplitude is a property of the efferent arm of the reflex (Maggi et al., *J. Pharmacol. Meth*., 15, 157, 1986; Maggi et al., *Brain Res..* 415, 1, 1987; Maggi et al., *Naun. Schmied. Arch. Pharmacol*., 332, 276, 1986; Maggi et al., *J. Urol*., 136, 696, 1986). In this model system, compounds that act mainly on the CNS (such as morphine) cause a reduction in the voiding frequency, whereas drugs that act at the level of the detrusor muscle lower the amplitude of the bladder contractions.

The results are tabulated in Tables 4 and 5 below.

**TABLE 4**

| **Effects on rhythmic bladder voiding contractions after intravenous administration.** | | | | | |
|---|---|---|---|---|---|
| *Data represent the mean values ± S. E. of the number of contractions observed before and after the i. v. administration of the tested compounds, as well as the amplitude of the peaks recorded in animals showing a reduction of the frequency <50%. The ED50 (and 95% confidence limits) values were evaluated on the basis of a quantal criterion as described in the Methods*. | | | | | |

| COMPOUND | | FREQUENCY | | AMPLITUDE | |
|---|---|---|---|---|---|
| Dose | No. | No. contr./15 min | | mm Hg | |
| µg/kg | of | before | after | before | after |
| i.v. | rats | treatment | treatment | treatment | treatment |
| Compound A | | | | | |
| 1 | 10 | 11.7±1.0 | 11.6±1.3 | 25.4±2.0 | 23.1±1.9* |
| 3 | 10 | 11.5±0.7 | 9.1±1.0* | 25.1±2.2 | 21.7±2.0* |
| 10 | 10 | 11.5±1.5 | 5.9±1.6* | 26.0±3.8 | 22.3±3.8 |
| 30 | 10 | 11.8±0.7 | 3.8±0.7* | 28.5±2.5 | 25.0±4.0 |
| 100 | 10 | 12.0±0.9 | 4.1±1.1* | 28.0±4.6 | 25.7±2.9 |
| 300 | 10 | 9.5±0.6 | 2.2±0.5* | n.c. | n.c. |
| ED₅₀(µg/kg) | | 5 (3 ö 8) | | n.a. | |
| Compound B | | | | | |
| 0.3 | 6 | 10.7±1.4 | 11.3±1.7 | 31.0±3.7 | 27.7±4.1 |
| 1 | 6 | 11.7±1.5 | 8.7±1.3 | 26.5±4.3 | 22.7±5.1* |
| 3 | 6 | 9.7±0.8 | 4.7±1.1* | 33.7±2.4 | 25.3±1.2 |
| 10 | 6 | 11.7±1.7 | 4.2±0.9* | 19.0 | 17.0 |
| 30 | 6 | 12.0±1.1 | 4.5±1.4 | n.c. | n.c. |
| ED₅₀(µg/kg) | | 1 (0.6 ö 2) | | n.a. | |
| Flavoxate | | | | | |
| 300 | 5 | 9.2±1.2 | 8.8±1.7 | 25.9±1.3 | 24.1±1.8 |
| 1000 | 17 | 10.1±0.7 | 8.6±0.9 | 26.1±2.3 | 25.0±2.2 |
| 3000 | 21 | 10.7±0.7 | 6.5±0.7* | 18.9±0.9 | 16.6±1.2 |
| 10000 | 20 | 11.3±0.8 | 5.5±0.6* | 19.9±1.6 | 19.2±1.5 |
| ED₅₀(µg/kg) | | 2650(1430 ö 4910) | | n. a. | |
| Oxybutynin | | | | | |
| 30 | 6 | 14.8±1.9 | 15.5±2.3 | 26.7±2.7 | 23.3±2.5 |
| 100 | 6 | 13.3±1.1 | 14.7±1.0 | 25.0±3.7 | 18.7±2.7* |
| 300 | 12 | 10.1±0.7 | 8.8±0.8 | 20.9±1.7 | 13.6±0.8* |
| 1000 | 13 | 9.7±0.8 | 9.5±0.8 | 20.3±2.4 | 11.8±1.2* |
| 3000 | 13 | 10.0±0.7 | 9.9±1.5 | 18.0±1.4 | 10.8±1.0 |
| ED₅₀(µg/kg) | | n.a. | | 240 (140 ö 400) | |
| NAN-190^{(A)} | | | | | |
| 30 | 10 | 13.5±1.2 | 11.8±1.5 | 30.2±3.7 | 26.1±3.1* |
| 100 | 10 | 13.6±1.0 | 6.4±1.1* | 22.5±1.7 | 17.3±2.9 |
| 300 | 11 | 11.8±1.1 | 6.6±1.1* | 24.1±1.8 | 17.6±1.7* |
| ED₅₀(µg/kg) | | 46 (23 ö 92) | | n.a. | |
| * = p≤0.01 (Student's *t* test for paired data) n.c. = not calculated n.a. = not active on the parameter A) = higher doses were not tested because of the high toxicity and low solubility of this compound. | | | | | |

**TABLE 5**

| **Effects on rhythmic bladder voiding contractions after intravenous administration.** | | | |
|---|---|---|---|
| *Data represent the mean values ± S. E. of the duration of bladder quiescence (disappearance time of contractions in min). The ED*_{*10min*} *values represent the extrapolated dose inducing 10 min of disappearance of the contractions.* | | | |

| COMPOUND | | BLADDER CONTRACTIONS | |
|---|---|---|---|
| Dose µg/kg i. v. | No. of rats | Disappearance time (min) | |
| Compound A | | | |
| 1 | 10 | 1.34 ± | 0.23 |
| 3 | 10 | 2.15 ± | 0.42 |
| 10 | 10 | 8.13 ± | 1.90 |
| 30 | 10 | 8.87 ± | 1.08 |
| 100 | 10 | 12.56 ± | 2.07 |
| 300 | 10 | 13.37 ± | 1.83 |
| ED₁₀ₘᵢₙ(µg/kg) | | 37 (18 ö 77) | |

| Compound B | | | |
|---|---|---|---|
| 0.3 | 6 | 1.10 ± | 0.16 |
| 1 | 6 | 4.33 ± | 1.30 |
| 3 | 6 | 7.58 ± | 2.15 |
| 10 | 6 | 10.00 ± | 0.92 |
| 30 | 6 | 8.85 ± | 1.53 |
| ED₁₀ₘᵢₙ(µg/kg) | | 9 (3 ö 24) | |

| Flavoxate | | | |
|---|---|---|---|
| 300 | 5 | 1.70 ± | 0.60 |
| 1000 | 17 | 3.04 ± | 0.96 |
| 3000 | 21 | 5.30 ± | 1.00 |
| 10000 | 20 | 8.25 ± | 1.90 |
| ED₁₀ₘᵢₙ(µg/kg) | | > 10000 | |
| NAN-190^{(A)} | | | |
| 30 | 10 | 1.80 ± | 0.52 |
| 100 | 10 | 6.34 ± | 1.18 |
| 300 | 11 | 5.47 ± | 1.93 |
| ED₁₀ₘᵢₙ(µg/kg) | | > > 300 | |
| A) = higher doses were not tested because of the high toxicity and low solubility of this compound. | | | |

Compound B, after intravenous administration, dose dependently inhibited the frequency of the rhythmic bladder voidings and also reduced amplitude to some extent. Compound A, after intravenous administration, dose dependently inhibited the frequency of the rhythmic bladder voidings with no effect on their amplitude. The maximal change of this parameter, in fact, was about 13% and no dose-dependence was observed. Of the test compounds shown, Compound B was the most potent, being 46- and 2650-fold more active than NAN-190 and flavoxate, respectively. Compound A was 9- and 530-fold more active than NAN-190 and flavoxate, respectively.

By contrast, oxybutynin was only effective at reducing the amplitude of the contractions, confirming that its effects are due to a complete inhibition of the muscarinic receptors in the bladder.

The compounds that reduced the contraction frequency induced a complete and transient disappearance of contractions for a time period that was directly proportional to the administered dose (Table 5).

Tables 4 and 5 also illustrate the effects on volume-induced bladder contractions of flavoxate, a drug widely utilized in clinical therapy for bladder dysfunctions.

Administration of this drug resulted in suppression of bladder contractions. The mean disappearance time observed after administration of the highest tested dose (10,000 µg/kg i.v.) was 8.25 ± 1.90 min. NAN-190 at the highest tested doses of 100-300 µg/kg gave a maximum disappearance time ranging from 5.5 to 6.3 min. (higher doses were not tested because of the high toxicity and low solubility of this compound).

These results indicate that Compound B and Compound A are potent compounds in reducing the frequency of the voiding contractions when compared to tlavoxate and NAN-190 in terms of both absolute potency (ED₅₀) and disappearance time (ED₁₀ₘᵢₙ). Their mechanism of action appears to be different from that of oxybutynin, a peripheral antimuscarinic. Furthermore, their effects appeared at very low doses.

### Example 7

### Effect of 5-HT_{1A} Receptor Antagonists on Cystometric Parameters in the Conscious Rat

### A. Methods:

Male Sprague Dawley rats (Crl: CD° BR) weighing 250-350 g were used. The animals were housed with free access to food and water and maintained on a forced 12 h alternating light-dark cycle at 22-24°C for at least one week, except during performance of the experiment.

To quantify urodynamic parameters in conscious rats, cystometrographic studies were performed using procedures described in Pietra et al., *IRCS Med: Sci*., 14, 992, 1986; and Guarneri et al., *Pharmacol. Res*., 24, 175, 1991.

Male rats were anaesthetized with nembutal (30 mg/kg) and chloral hydrate (125 mg/kg) i.p. and were placed in a supine position. An approximately 10 mm long midline incision was made in the shaved and cleaned abdominal wall. The urinary bladder was gently freed from adhering tissues, emptied, and then cannulated, via an incision at the dome, with a polyethylene cannula (Portex PP30), which was permanently sutured with silk thread. The cannula was exteriorized through a subcutaneous tunnel in the retroscapular area, where it was connected with a plastic adapter to avoid the risk of removal by the animal. For intravenous (i.v.) injection of test compounds, a PE 50 polyethylene tubing filled with physiological saline was inserted into the jugular vein and exteriorized in the retroscapular area.

Since cystometrographic parameters have been reported to be influenced by the time elapsed after catheter implantation Yaksh et al. *Amer. J. Physiol*., 251, R1177, 1986 the rats were utilized exclusively one day after implantation.

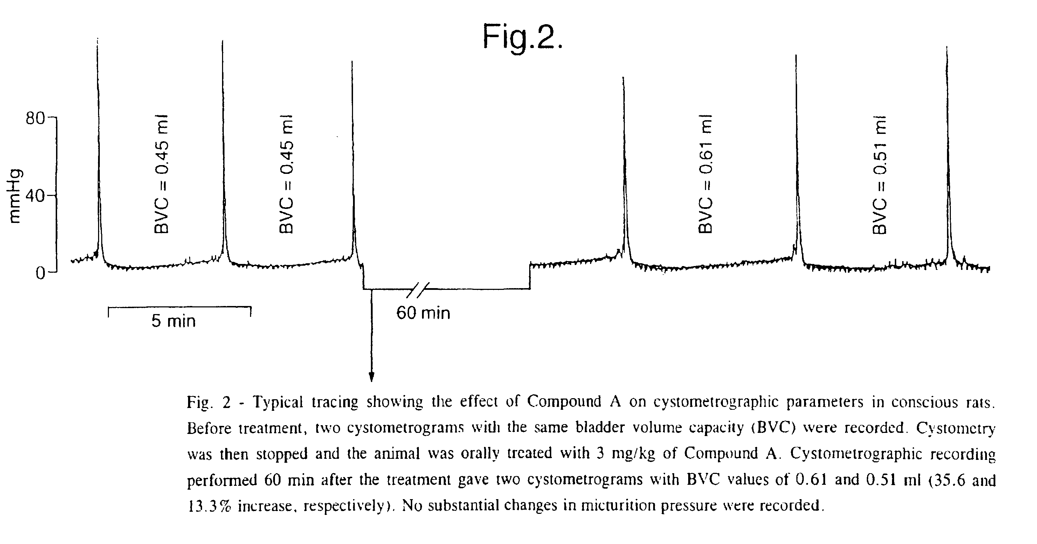

On the day of the experiment, the rats were placed in Bollman's cages; after a stabilization period of 20 min, the free tip of the bladder catheter was connected through a T-shaped tube to a pressure transducer (Bentley T 800/Marb P 82) and to a peristaltic pump (Gilson minipuls 2) for a continuous infusion, at the constant rate of 0.1 ml/min, of saline solution into the urinary bladder. The intraluminal pressure signal during infusion was continuously recorded on a polygraph (Battaglia Rangoni KO 380 with ADC1/T amplifier). Two urodynamic parameters were evaluated: bladder volume capacity (BVC) and micturition pressure (MP). BVC (in ml) is defined as the minimum volume infused after which detrusor contraction (followed by micturition) occurs. MP (in mmHg) is defined as the maximal intravesical pressure induced by the contraction of detrusor during micturition. Basal BVC and MP values were calculated as the means of the first two recorded cystometrograms. At this point, the infusion was interrupted and the test compounds were administered. Fifteen minutes after intravenous administration, or one hour after oral drug administration, two additional cystometrograms were recorded in each animal and the mean values of the two cystometrographic parameters were calculated. A typical tracing is shown in Figure 2, where the effects of 3 mg/kg p.o. of Compound A are shown.

The statistical significance of the differences in urodynamic parameter values was evaluated by Student's *t* test for paired data. Only changes showing a probability P<0.01 were considered to be significant.

### B. Results:

The effects on urodynamic parameters in conscious rats after i.v. administration of different doses of Compound A and the reference compounds are summarized in Tables 6 and 7.

**TABLE 6**

| **Effects on cystometrogram in conscious rats.** | | | | |
|---|---|---|---|---|
| *Data represent the mean ± S. E. values (ml) of bladder volume capacity (BVC), before and 15 min after i. v. injection of the compounds*. | | | | |

| COMPOUND | | BVC | | |
|---|---|---|---|---|
| Dose | No. of | before | after | % |
| µg/kg i.v. | rats | treatment | treatment | change |
| CONTROL | | | | |
| vehicle | 12 | 0.50±0.09 | 0.43±0.06 | -17 |
| Compound A | | | | |
| 100 | 8 | 0.65±0.06 | 0.66±0.08 | +4 |
| 300 | 9 | 0.47±0.05 | 0.63±0.06* | +32 |
| 1000 | 20 | 0.64±0.05 | 0.83±0.07* | +29 |
| 2000 | 10 | 0.48±0.04 | 0.63±0.05* | +32 |
| Flavoxate | | | | |
| 300 | 17 | 0.76±0.11 | 0.87±0.11 | +14 |
| 1000 | 14 | 0.88±0.15 | 1.11±0.16* | +26 |
| 3000 | 18 | 0.77±0.08 | 1.07±0.12* | +39 |
| Oxybutynin | | | | |
| 100 | 13 | 0.82±0.15 | 0.89±0.18 | +9 |
| 300 | 12 | 0.83±0.13 | 0.83±0.12 | 0 |
| 1000 | 12 | 0.94±0.19 | 1.00±0.18 | +7 |
| NAN-190^{(A)} | | | | |
| 30 | 8 | 0.74±0.09 | 0.78±0.10 | +6 |
| 100 | 8 | 0.68±0.10 | 0.76±0.10 | +12 |
| 300 | 8 | 0.62±0.06 | 0.61±0.06 | -1 |

| | | | | |
|---|---|---|---|---|
| * = p ≤ 0.01 (Student's *t* test for paired data) | | | | |
| A) = higher doses were not tested because of the high toxicity and low solubility of this compound. | | | | |

**TABLE 7**

| **Effects on cystometrogram in conscious rats.** | | | | |
|---|---|---|---|---|
| *Data represent the mean values ± S.E. (mmHg) of micturition pressure (MP), before and 15 min after i. v. injection of the compounds*. | | | | |

| COMPOUND | | MP | | |
|---|---|---|---|---|
| Dose | No. of | before | after | % |
| µg/kg i.v. | rats | treatment | treatment | change |
| CONTROL | | | | |
| vehicle | 12 | 91.3±9.2 | 87.9± 9.9 | -4 |
| Compound A | | | | |
| 100 | 8 | 93.0±8.3 | 83.8±8.7 | -10 |
| 300 | 9 | 78.7±5.8 | 70.0±4.1 | -11 |
| 1000 | 20 | 104.6±6.4 | 91.0±6.3* | -13 |
| 2000 | 10 | 101.8±10.9 | 81.5±14.1 | -20 |
| Flavoxate | | | | |
| 300 | 17 | 89.2±10.7 | 95.0±10.9 | +7 |
| 1000 | 14 | 90.4±10.7 | 80.1±11.1 | -12 |
| 3000 | 18 | 72.6±9.3 | 75.2±9.5 | +4 |
| Oxybutynin | | | | |
| 100 | 13 | 95.2±9.2 | 77.4±10.3* | -19 |
| 300 | 12 | 82.3±8.7 | 50.5±6.3* | -39 |
| 1000 | 12 | 110.9±10.2 | 29.6±5.6* | -73 |
| NAN-190^{(A)} | | | | |
| 30 | 8 | 99.4±10.1 | 104.6±9.7 | +5 |
| 100 | 8 | 93.8±11.5 | 82.5±9.2 | -12 |
| 300 | 8 | 86.6±10.3 | 88.4±11.8 | +2 |

| | | | | |
|---|---|---|---|---|
| * = p ≤0.01 (Student's *t* test for paired data) | | | | |
| A) = higher doses were not tested because of the high toxicity and low solubility of this compound. | | | | |

The administration of Compound A induced constant and significant increases of the BVC. Flavoxate (1000-3000 µg/kg) also induced increases in BVC, and the differences between basal and after treatment values were statistically significant (Table 6).

Oxybutynin was inactive on BVC (Table 6), but induced very consistent, significant and dose-related reductions of MP (the approximate ED₅₀ value was 400 µg/kg), in contrast to Compound A and flavoxate which were inactive on this parameter (Table 7). NAN-190 was devoid of significant effects on both parameters up to the highest administrable dose of 300 µg/kg.

The effects of these compounds after oral administration were also tested. The results are shown in Tables 8 and 9 below.

**TABLE 8**

| **Effects on cystometrogram in conscious rats.** | | | | |
|---|---|---|---|---|
| *Data represent the mean values ± S.E. (ml) of bladder volume capacity (BVC), before and 1 hour after oral administration of the compounds.* | | | | |

| COMPOUND | | BVC | | |
|---|---|---|---|---|
| Dose | No. of | before | after | % |
| mg/kg p.o. | rats | treatment | treatment | change |
| CONTROL | | | | |
| vehicle | 11 | 0.64±0.10 | 0.73 ±0.13 | +14 |
| Compound A | | | | |
| 1 | 10 | 0.52±0.07 | 0.60±0.08 | + 15 |
| 3 | 10 | 0.67±0.07 | 0.91±0.10* | +35 |
| 10 | 10 | 0.54±0.06 | 0.73 ±0.10* | +37 |
| Oxybutynin | | | | |
| 1 | 8 | 0.56±0.11 | 0.74±0.11* | +31 |
| 3 | 8 | 0.54±0.07 | 0.63±0.13 | +18 |
| 10 | 8 | 0.55±0.08 | 0.70±0.11 | +27 |
| NAN-190 | | | | |
| 10 | 10 | 0.54±0.08 | 0.46±0.07 | -14 |
| 30 | 10 | 0.71±0.09 | 0.60±0.09 | -15 |

| | | | | |
|---|---|---|---|---|
| * = p ≤0.01 (Student's *t* test for paired data) | | | | |

**TABLE 9**

| **Effects on cystometrogram in conscious rats.** | | | | |
|---|---|---|---|---|
| *Data represent the mean values ± S. E. (mmHg) of micturition pressure (MP), before and 1 hour after oral administration of the compounds*. | | | | |

| Compound | | MP | | |
|---|---|---|---|---|
| Dose | No. of | before | after | % |
| mg/kg p.o. | rats | treatment | treatment | change |
| CONTROL | | | | |
| vehicle | 11 | 84.1±10.1 | 73.3±11.0 | -13 |
| Compound A | | | | |
| 1 | 10 | 96.0±8.4 | 93.7±7.2 | -2 |
| 3 | 10 | 112.5±6.5 | 107.6±9.2 | -4 |
| 10 | 10 | 90.2±7.1 | 86.6±7.6 | -4 |
| Oxybutynin | | | | |
| 1 | 8 | 92.1±13.3 | 77.3±9.8 | -16 |
| 3 | 8 | 82.1±5.1 | 42.1±5.1* | -49 |
| 10 | 8 | 98.3±9.0 | 31.8±3.9* | -68 |
| NAN-190 | | | | |
| 10 | 10 | 106.1±10.4 | 90.8±12.5 | -14 |
| 30 | 10 | 105.1±10.5 | 95.8±15.3 | -9 |

| | | | | |
|---|---|---|---|---|
| * = p ±0.01 (Student's *t* test for paired data) | | | | |

Compound A produced a significant increase of BVC after oral administration of 3 mg/kg, and no changes in MP values were detected. Oxybutynin caused a significant increase of the BVC after oral administration at the lowest utilized dose (1 mg/kg), and produced a dose-related reduction of the MP values that was consistent and significant with 3 and 10 mg/kg dose-levels (approximate ED₅₀ value was 4 mg/kg). NAN-190 was inactive after oral administration at doses up to 30 mg/kg, a dose 10-fold higher than the minimal effective dose of Compound A.

These results were consistent with those obtained in anaesthetized rats as described in Example 6 above. Compound A was found to be active in increasing the BVC without affecting bladder contractility (MP), in contrast to oxybutynin. Compound A was also found to be active after both i.v. and oral administration, in contrast to NAN-190 which was inactive after i.v. or oral administration of doses up to 10-fold higher than those used for Compound A.

The above results show that compounds endowed with antagonistic activity at pre- and post-synaptic 5-HT_{1A} receptors and devoid of significant affinity for the α₁-adrenergic receptors are unexpectedly endowed with a potent pharmacological activity on the lower urinary tract. In particular, these compounds are able to inhibit the micturition reflex and to increase the period between micturition without impairing the capability of detrusor to have effective voidings once the micturition threshold has been reached. This is important since the drugs currently used for treatment of urinary incontinence (mainly anticholinergics) decrease efficiency of micturition, as shown for oxybutynin in Example 7, when the micturition pressure is reduced, and causes an increase of residual volume, due to compromission of bladder contractile force.

## Claims

1. Use of a compound which
(a) binds to a cloned human 5-HT_{1A} receptor with an affinity of at least 10⁻⁷ M,
(b) binds to a rat 5-HT_{1A} receptor with an affinity at least 50-fold stronger than the affinity with which the compound binds to a rat α₁-adrenergic receptor, and
(c) exhibits 5-HT_{1A} receptor antagonist activity on both mouse pre-synaptic and rat post-synaptic 5-HT_{1A} receptors
for the preparation of a medicament for the treatment of lower urinary tract disorders in mammals.

2. Use according to claim 1 of a compound having the general formula I 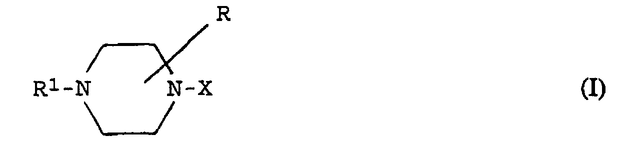 wherein
R represents a hydrogen atom or a (C₁-C₄)alkyl group;
R¹ represents an aryl, nitrogen containing heteroaryl or bicyclic heteroaryl group;
X represents one of the groups 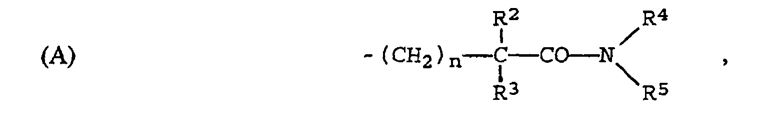 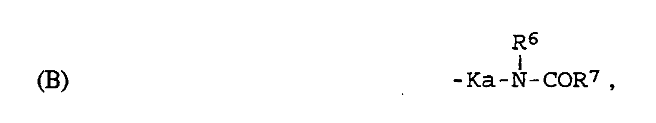 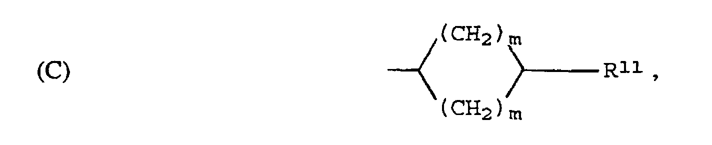 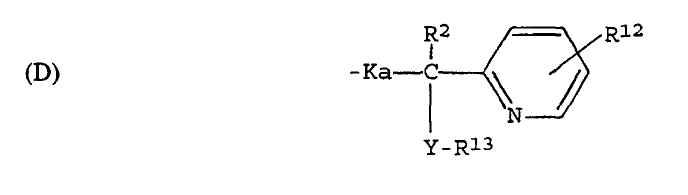 and 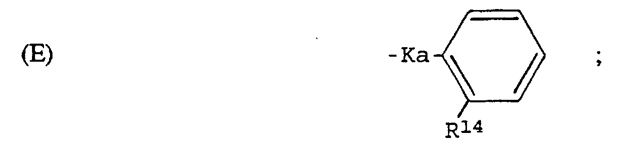 n is 1 or 2;
m is 1, 2 or 3;
R² represents a hydrogen atom or a (C₁-C₄)alkyl group;
R³ represents an aryl or aryl-(C₁-C₄)alkyl group;
R⁴ represents a hydrogen atom or a C₁-C₃ alkyl group;
R⁵ represents a hydrogen atom or a C₁-C₃ alkyl, C₃-C₁₂ cycloalkyl or cycloalkyl-(C₁-C₄)alkyl group; or R⁴ and R⁵ together with the nitrogen atom to which they are attached represent a 1-azetidinyl, 1-pyrrolidinyl, piperidino, 1-perhydroazepinyl, morpholino, or 1-piperazinyl group, each optionally substituted by a (C₁-C₄)alkyl, aryl or aryl-(C₁-C₄)alkyl group;
R⁶ represents a monocyclic or bicyclic heteroaryl group;
R⁷ represents a hydrogen atom, a (C₁-C₄)alkyl, cycloalkyl, cycloalkenyl, cycloalkyl-(C₁-C₄)alkyl, aryl, aryl-(C₁-C₄)alkyl, heteroaryl or heteroaryl-(C₁-C₄)alkyl group, or a group -NR8R9 or OR¹⁰;
R⁸ represents a hydrogen atom or a (C₁-C₄)alkyl, aryl or aryl-(C₁-C₄)alkyl group;
R⁹ represts a hydrogen atom or a (C₁-C₄)alkyl, -CO-(C₁-C₄)alkyl, aryl, -CO-aryl, aryl-(C₁-C₄)alkyl, cycloalkyl or cycloalkyl-(C₁-C₄)alkyl group; or R⁸ and R⁹ together with the nitrogen atom to which they are attached represent a saturated heterocyclic group which may contain an additional heteroatom;
R¹⁰ represents a (C₁-C₄)alkyl, cycloalkyl, cycloalkyl-(C₁-C₄)alkyl, aryl, aryl-(C₁-C₄)alkyl, heteroaryl or heteroaryl-(C₁-C₄)alkyl group;
R¹¹ represents an aryl or nitrogen containing heteroaryl group;
R¹² represents a hydrogen atom or a (C₁-C₄)alkyl group;
R¹³ represents a hydrogen atom or a C₁-C₃ alkyl, C₃-C₁₂ cycloalkyl or cycloalkyl-(C₁-C₄)alkyl group;
R¹⁴ represents an aryl group;
Ka represents a C₂-C₄ alkylene group optionally substituted by one or more (C₁-C₄)alkyl groups; and
Y represents a carbonyl, alkylene, hydroxyalkylene or hydroxycycloalkylene group or a group -S(O)ₒ; where o = 0 to 2.

3. Use according to claim 1 of 1-[N-cyclohexylcarbonyl-N-(2-pyridyl)-- 2-aminoethyl)-4-(2-methoxyphenyl)-piperazine.

4. Use according to claim 1 of 1-[N-cyclohexylcarbonyl-N-(2-pyridyl)--2-aminoethyl)-4-(4-indolyl)-piperazine.

## Patentansprüche

1. Verwendung einer Verbindung, welche
(a) an einen klonierten menschlichen 5-HT_{1A} Rezeptor mit einer Affinität von mindestens 10⁻⁷ M bindet,
(b) an einen 5-HT_{1A} Rezeptor aus Ratte mit einer mindestens 50-fach stärkeren Affinität bindet als der Affinität, mit der die Verbindung an einen Ratten α₁-adrenergen Rezeptor bindet, und
(c) 5-HT_{1A} Rezeptor antagonistische Aktivität sowohl hinsichtlich präsynaptischer 5-HT_{1A} Rezeptoren aus Maus als auch post-synaptischer 5-HT_{1A} Rezeptoren aus Ratten aufweist,
zur Herstellung eines Medikamentes für die Behandlung von Erkrankungen des unteren Hamweges bei Säugetieren.

2. Verwendung nach Anspruch 1 einer Verbindung mit der allgemeinen Formel I 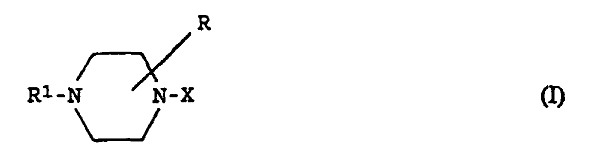 worin
R für ein Wasserstoffatom oder eine (C₁-C₄)Alkylgruppe steht;
R¹ für eine Arylgruppe, eine Stickstoff enthaltende Heteroarylgruppe
oder eine bizyklische Heteroarylgruppe steht;
X für eine der Gruppen 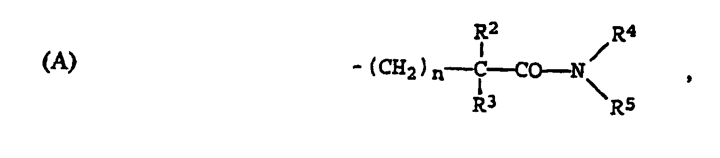 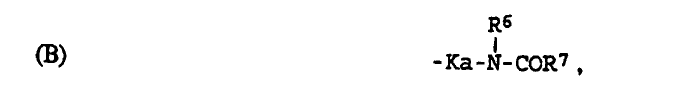 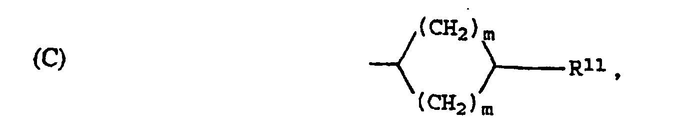 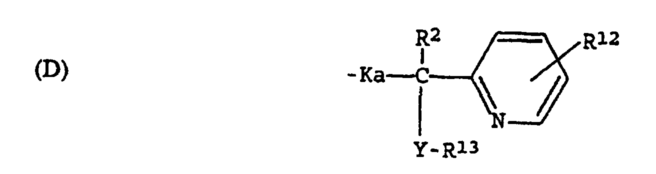 und 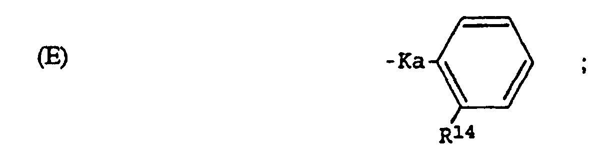 steht, wobei
n 1 oder 2 ist;
m 1, 2 oder 3;
R² für ein Wasserstoffatom oder eine (C₁-C₄)Alkylgruppe steht;
R³ für eine Arylgruppe oder eine Aryl-(C₁-C₄)Alkylgruppe steht;
R⁴ für ein Wasserstoffatom oder eine C₁-C₃ Alkylgruppe steht;
R⁵ für ein Wasserstoffatom oder eine C₁-C₃ Alkyl, C₃-C₁₂ Cycloalkyl oder Cycloalkyl-(C₁-C₄)Alkylgruppe steht, oder R₄ oder R₅ zusammen mit dem Stickstoffatom, an welches sie gebunden sind, für eine 1-Azetidinyl, 1-pyrrolidinyl, Piperidino, 1-Perhydroazepinyl, Morpholino, oder 1-Piperazinyl-Gruppen stehen, wobei jede gegebenenfalls durch eine (C₁-C₄)Alkyl, Aryl oder Aryl-(C₁-C₄)Alkylgruppe substituiert sein kann;
R⁶ für eine monozyklische oder eine bizyklische Heteroarylgruppe steht; R⁷ für ein Wasserstoffatom, ein (C₁-C₄)Alkyl, Cycloalkyl, Cycloalkenyl, Cycloalkyl-(C₁-C₄)Alkyl, Aryl, Aryl-(C₁-C₄)Alkyl, Heteroaryl oder Heteroaryl-(C₁-C₄)Alkylgruppe, oder eine NR⁸R⁹ oder OR¹⁰-Gruppe steht;
R⁸ für ein Wasserstoffatom oder eine (C₁-C₄)Alkyl, Aryl oder Aryl-(C₁-C₄)Alkylgruppe steht;
R⁹ für ein Wasserstoffatom oder eine (C₁-C₄)Alkyl, -CO-(C₁-C₄)Alkyl, Aryl, -CO-Aryl, Aryl-(C₁-C₄)Alkyl, Cycloalkyl oder Cycloalkyl-(C₁-C₄)Alkylgruppe steht; oder R⁸ und R⁹ zusammen mit dem Stickstoffatom, an welches sie gebunden sind, für eine gesättigte heterocyclische Gruppe steht, welche ein zusätzliches Heteroatom enthalten kann;
R¹⁰ für eine (C₁-C₄)Alkyl, Cycloalkyl, Cycloalkyl-(C₁-C₄)Alkyl, Aryl, Aryl-(C₁-C₄)Alkyl, Heteroaryl oder Heteroaryl-(C₁-C₄)Alkylgruppe steht; R¹¹ für eine Aryl oder stickstoffenthaltende Heteroarylgruppe steht;
R¹² für ein Wasserstoffatom oder ein (C₁-C₄)Alkylgruppe steht;
R¹³ für ein Wasserstoffatom oder eine C₁-C₃ Alkyl, C₃-C₁₂ Cycloalkyl oder Cycloalkyl-(C₁-C₄)Alkylgruppe steht;
R¹⁴ für eine Arylgruppe steht;
Ka für eine C₂-C₄ Alkylengruppe steht, welche gegebenenfalls durch eine oder mehrere (C₁-C₄)Alkylgruppen substituiert sein kann; und
Y für eine Carbonyl, Alkylen, Hydroxyalkylen oder Hydroxycycloalkylengruppe oder eine -S(O)ₒ-Gruppe steht; wobei o = 0 bis 2 ist.

3. Verwendung nach Anspruch 1 von 1-[N-cyclohexylcarbonyl-N-(2-pyridyl)-2-aminoethyl)-4-(2-methoxyphenyl)-Piperazin.

4. Verwendung nach Anspruch 1 von 1-[N-cyclohexylcarbonyl-N-(2-pyridyl)-2-aminoethyl)-4-(4-indolyl)-Piperazin.

## Revendications

1. Utilisation d'un composé qui
(a) se lie à un récepteur 5-HT_{1A} humain cloné avec une affinité d'au moins 10⁻⁷ M,
(b) se lie à un récepteur 5-HT_{1A} de rat avec une affinité au moins 50 fois plus forte que l'affinité avec laquelle le composé se lie à un récepteur α₁-adrénergique de rat, et
(c) présente une activité d'antagoniste des récepteurs 5-HT_{1A} sur les récepteurs 5-HT_{1A} tant présynaptiques de souris que postsynaptiques de rat
pour la préparation d'un médicament pour le traitement d'affections du bas appareil urinaire chez des mammifères.

2. Utilisation selon la revendication 1 d'un composé ayant la formule générale I 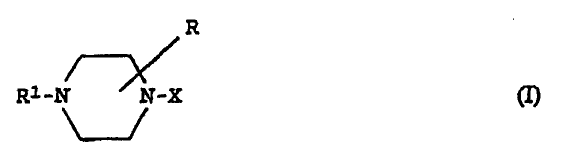 dans laquelle
R représente un atome d'hydrogène ou un groupe alkyle en C₁ à C₄ ;
R¹ représente un groupe aryle, hétéroaryle ou hétéroaryle bicyclique contenant de l'azote ;
X représente l'un des groupes 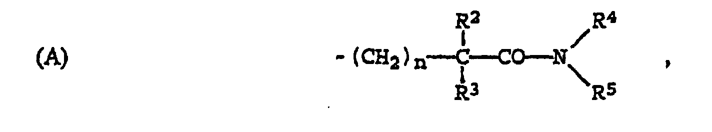 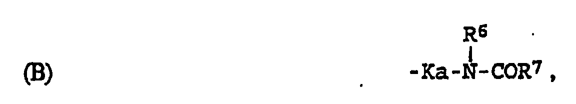 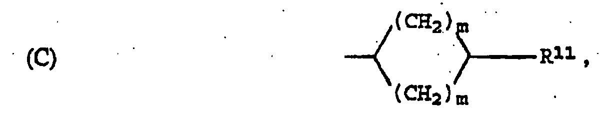 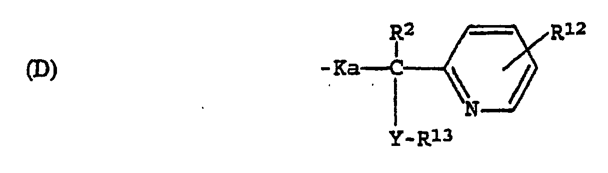 et 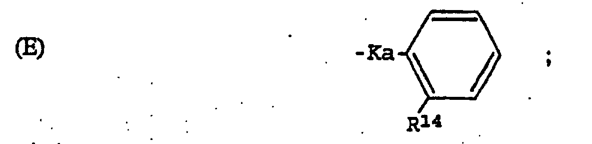 n est 1 ou 2 ;
m est 1, 2 ou 3 ;
R² représente un atome d'hydrogène ou un groupe alkyle en C₁ à C₄ ;
R³ représente un groupe aryle ou aryl-alkyle en C₁ à C₄ ;
R⁴ représente un atome d'hydrogène ou un groupe alkyle en C₁ à C₃ ;
R⁵ représente un atome d'hydrogène ou un groupe alkyle en C₁ à C₃, cycloalkyle en C₃ à C₁₂ ou cycloalkyl-alkyle en C₁ à C₄ ; ou R⁴ et R⁵, avec l' atome d'azote auquel ils sont attachés, représentent un groupe 1-azétidinyle, 1-pyrrolidinyle, pipéridino, 1-perhydroazépinyle, morpholino ou 1-pipérazinyle, chacun facultativement substitué par un groupe alkyle en C₁ à C₄, aryle ou aryl-alkyle en C₁ à C₄ ;
R⁶ représente un groupe hétéroaryle monocyclique ou bicyclique ;
R⁷ représente un atome d'hydrogène, un groupe alkyle en C₁ à C₄, cycloalkyle, cycloalcényle, cycloalkyl-alkyle en C₁ à C₄, aryle, aryl-alkyle en C₁ à C₄, hétéroaryle ou hétéroaryl-alkyle en C₁ à C₄, ou un groupe -NR⁸R⁹ ou OR¹⁰ ;
R⁸ représente un atome d'hydrogène ou un groupe alkyle en C₁ à C₄, aryle ou aryl-alkyle en C₁ à C₄ ;
R⁹ représente un atome d'hydrogène ou un groupe alkyle en C₁ à C₄, -CO-alkyle en C₁ à C₄, aryle, -CO-aryle, aryl-alkyle en C₁ à C₄, cycloalkyle ou cycloalkyl-alkyle en C₁ à C₄ ; ou R⁸ et R⁹, avec l'atome d'azote auquel ils sont attachés, représentent un groupe hétérocyclique saturé qui peut contenir un hétéroatome supplémentaire ;
R¹⁰ représente un groupe alkyle en C₁ à C₄, cycloalkyle, cycloalkyl-alkyle en C₁ à C₄, aryle, aryl-alkyle en C₁ à C₄, hétéroaryle ou hétéroaryl-alkyle en C₁ à C₄ ;
R¹¹ représente un groupe aryle, ou hétéroaryle contenant de l'azote ;
R¹² représente un atome d'hydrogène ou un groupe alkyle en C₁ à C₄ ;
R¹³ représente un atome d'hydrogène ou un groupe alkyle en C₁ à C₃, cycloalkyle en C₃ à C₁₂ ou cycloalkyl-alkyle en C₁ à C₄ ;
R¹⁴ représente un groupe aryle ;
Ka représente un groupe alkylène en C₂ à C₄ facultativement substitué par un ou plusieurs groupes alkyles en C₁ à C₄ ; et
Y représente un groupe carbonyle, alkylène, hydroxyalkylène ou hydroxycycloalkylène ou un groupe -S(O)ₒ ; où o = 0 à 2.

3. Utilisation selon la revendication 1 de 1-[N-cyclohexylcarbonyl-N-(2-pyridyl)-2-aminoéthyl)-4-(2-méthoxyphényl)pipérazine.

4. Utilisation selon la revendication 1 de 1-[N-cyclohexylcarbonyl-N-(2-pyridyl)-2-aminoéthyl)-4-(4-indolyl)-pipérazine.
